# EUROPEAN PATENT APPLICATION

(11) **EP 2 319 545 A1**
(43) Date of publication of application: **11.05.2011**
(21) Application number: 09252447.9
(22) Date of filing: 20.10.2009
(51) Int. Cl.: A61L 2/10, F02M 27/04, F02M 27/06

(54) **Fuel impurity reduction apparatus and method**

(71) Applicant: Pethybridge, Grant Thomas, Waitakere (NZ)
(72) Inventor: Pethybridge, Grant Thomas, Waitakere (NZ)
(74) Representative: Jones, Graham Henry

(57) **Abstract**

A fuel impurity reduction apparatus includes a sterilizing means, microbial contamination agitating means, operatively interconnected together to a supply of fuel whereby the fuel is pumped under pressure to and through the microbial contamination agitating means and past the sterilizing means which includes a UV light means, and the microbial contamination agitating means includes a passageway with magnetic means therein such that the fuel is treated to reduce any microbial contamination in the fuel. The fuel is pumped from a lower end of the sterilizing means to an upper end of the sterilizing means so that the sterilizing means is always completely surrounded by fuel when the sterilizing means is activated.

A method of treating the fuel is also included. The microbial contamination agitating means and sterilizing means can be combined in one housing with an upper and lower housing or they can be formed as spaced separate housings in one main housing which can then include pumping means and pressure switch.

## Description

The invention relates to a fuel impurity reduction apparatus and to a method of treating fuel. The invention is directed particularly but not solely towards reducing or eliminating or controlling impurities in fuels and related fluids such as for example diesel and oil, home heating oil, lubricating oil and aviation spirit.

### Background of Invention

As fuel is an organic hydrocarbon, liquid at room temperature, it can act as a medium for impurities such as plant and bacterial growth or microbial contamination. Sometimes this type of impurity can be termed a bug impurity. For example this fungal growth if allowed to grow, could cause problems for the use of the fuel for combustion.

The problems include blockage of fuel lines which can cause a reduction in fuel flow or even a sudden loss of engine power. Existing filter means can prevent or reduce this but do not reduce the presence of microscopic moisture. Existing methods require maintenance to clean or replace any filter elements and are expensive temporary solutions to the problem. Some methods of fuel treatment with sterilizing means causing problems with heat spots being formed which can be dangerous in proximity to fuel.

In this specification unless the contrary is expressly stated, where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

### Object of the Invention

It is an object of the invention to provide a fuel impurity reduction apparatus and method of treating fuel that ameliorates some of the disadvantages and limitations of the known art or at least provides the public with a useful choice.

### Summary of Invention

In a first aspect the invention resides in a fuel impurity reduction apparatus to treat fuel including a sterilizing means, microbial contamination agitating means, operatively interconnected together to a supply of fuel whereby the fuel is pumped under pressure to and through the microbial contamination agitating means and past the sterilizing means which includes a UV light means, and the microbial contamination agitating means includes a passageway with magnetic means therein such that the fuel is treated to reduce the incidence of microbial contamination wherein the fuel is pumped from a lower end of the sterilizing means to an upper end of the sterilizing means so that the sterilizing means is always completely surrounded by fuel when the sterilizing means is switched on or activated.

Preferably the apparatus includes a pumping means to enable the fuel to be pumped under pressure through the apparatus wherein the pumping means includes at least one pump.

Preferably the sterilizing means comprises at least one UV light means which is adapted to sterilize the fuel when it goes past where the UV light radiates to cause the sterilizing.

Preferably, the apparatus includes a pressure switching means which is adapted to sense or monitor any reduction in pressure of the flow to enable the flow to be stopped, and a manifold to allow a choice of filter means, microbial contamination agitating means or UV light means.

Preferably, the microbial contamination agitating means includes at least one housing with a passageway there-through for the fuel to pass through whereby there is at least one magnet within the passage.

Preferably, the magnets are removably affixed to both sides of the passageway.

Preferably, the UV light means includes a housing including an outer housing for the flow of fuel and an inner housing to hold a UV light means therein within the flow of fuel. Preferably, the outer housing of the housing includes a middle portion and end portions whereby the fuel only passes through the middle portion and end portions are fluidly sealed to not allow fuel therein and allow for the electrical connection of power means to power the UV light means.

Preferably, the fuel to be treated enters a lower end of one end of the middle portion of the outer housing and passes along the middle housing past the UV light means, to reach the opposite end of the middle portion and then exit there from.

Preferably the microbial contamination agitating means is directly combined with the sterilizing means in one separate apparatus wherein the housing for the microbial contamination agitating means is operatively connected to the housing for the sterilizing means.

Preferably, the UV light means is mounted above the pumping means whereby the outlet from the UV light means is located at the top of the UV light means.

Preferably, the apparatus includes a housing to house the pumping means, the UV light means and microbial contamination agitating means.

Preferably, the apparatus includes a control means operatively connected to any or all of the sterilizing means, microbial contamination agitating means, pump means and filter means.

Preferably, the filter means includes an inlet filter means located in a fuel supply means.

In a second aspect the invention resides in a method of treating fuel to reduce fuel impurities when operating a fuel impurity reduction apparatus which includes including a sterilizing means, microbial contamination agitating means, pumping means, pressure switching means and fuel line, operatively interconnected together to a supply of fuel whereby the fuel is pumped under pressure to and through the microbial contamination agitating means and past the sterilizing means which includes a UV light means, and the microbial contamination agitating means includes a passageway with magnetic means therein such that the fuel is treated to reduce any microbial contamination wherein the fuel is pumped from a lower end of the sterilizing means to an upper end of the sterilizing means, so that the sterilizing means is always completely surrounded by fuel when the sterilizing means is switched on or activated, wherein the steps of the method include:
- initiate control means - pumping begins and UV light means is switched on;
- fuel then goes through the filter means;
- pumping means pumps fuel from the filter means to the bug disrupting means
- alternatively the fuel can be pumped through another bug disrupting means;
- fuel passes up into and through UV light means;
- fuel passes into outer enclosed space of the UV light means;
- fuel passes UV light tube through both middle portions to be treated to reduce impurities and then the
- treated fuel passes out of UV light means outlet.

In a third aspect the invention resides in a microbial contamination agitating and sterilizing apparatus for treating fuel to reduce impurities, the apparatus includes a housing including a sterilizing means in one portion and microbial contamination agitating means in another portion, the sterilizing means including a UV light means in a first (or upper) housing and the microbial contamination agitating means including a second (or lower) housing with a passage way for the fuel and at least one magnet is located within the passage way, to allow fuel to travel through the passageway past the magnets and UV light, to be treated wherein there is a sealing means which allows the fuel to be fluidly sealed within the first housing and also allows UV light to penetrate there through from the second housing to treat the fuel by sterilizing and agitating the fuel.

Preferably the first housing has venting means to dissipate any heat build up from the UV light means.

Preferably the magnets are located in a middle portion of the passageway adjacent to the UV light means.

### Brief Description

The invention will now be described, by way of example only, by reference to the accompanying drawings:
**Figure 1** is a schematic representation of the fuel impurity reduction apparatus in accordance with a first preferred embodiment of the invention.
**Figure 2** is a front view of the apparatus in use.
**Figure 3** is a top plan view of the apparatus in use.
**Figure 4** is a left hand end view of the apparatus of figure 2.
**Figure 5** is a right hand end view of the apparatus of figure 2.

### UV lighting device

**Figure 6** is a top plan view of the UV light means.
**Figure 7** is a front cross sectional view of the UV light means of figure 6.
**Figure 8** is a cross sectional end view of the UV light means of figure 7.
**Figure 9** is a general view similar to figure 7 but showing location of close-up view.
**Figure 9a** is a close up cross section side view of one end of the UV light means.

### Microbial contamination agitating means

**Figure 10** is a cross sectional side view of microbial contamination agitating means.
**Figure 11** is a top plan view of the microbial contamination agitating means.
**Figure 12** is a cross sectional end view of the microbial contamination agitating means.

### Agitating and sterilizing means

**Figure 13** is cross sectional side view of a combined UV light and magnetic housing.
**Figure 14** is an end view of the combination of figure 13.
**Figure 15** is a cross sectional end view of the combination of figure 13.
**Figure 16** is a top view of the combination of figure 13

### Other examples

**Figure 17** is a schematic cross sectional side of view of a tank immersion fuel impurity device.
**Figures 18-21** are schematic views of an example of another version of the fuel impurity reduction apparatus.

### Description of Drawings

The following description will describe the invention in relation to preferred embodiments of the invention, namely a fuel impurity reduction apparatus. The invention is in no way limited to these preferred embodiments as they are purely to exemplify the invention only and that possible variations and modifications would be readily apparent without departing from the scope of the invention.

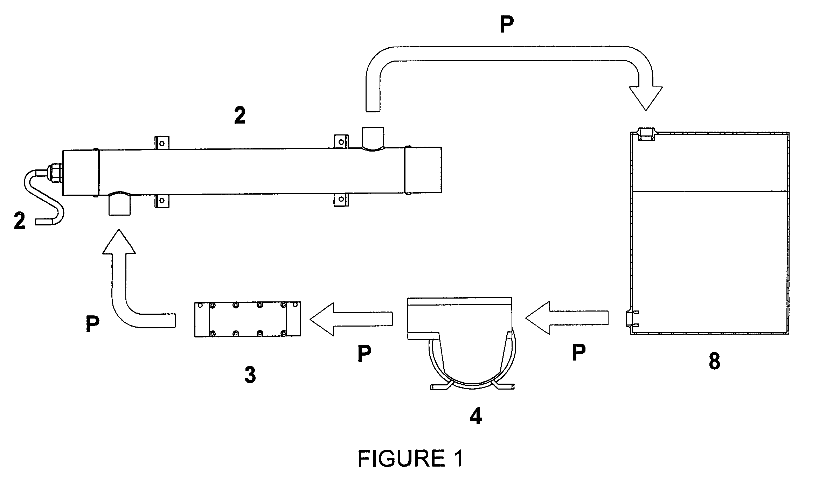

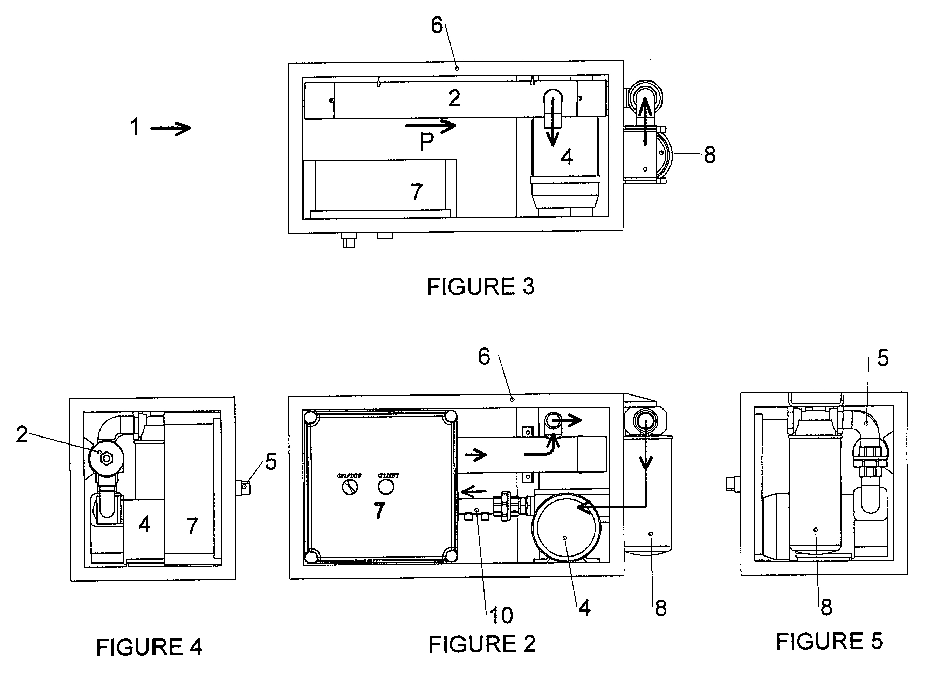

As shown in figures 1-21 there is fuel impurity reduction apparatus (1) which includes the following components of a sterilizing means (2) in the form of an ultra-violet (UV) light means, a fuel or microbial contamination agitating means (3), pumping means (4) operatively connected together by connection means such as by piping (5) which can be *enclosed within a suitable housing (6), The apparatus (1) can include a control means (7) as shown in figures 2-5 which can be operated with appropriate hardware and software such as programming which can include manual or automatic control eg by gate valves or wires or by wireless technology. The piping (5) can be termed a fuel line which is any tubular structure adapted to carry a fuel.

Also apparatus (1) is operatively connected to a fuel supply means (8) and power means (9). The fuel supply means (8) includes a fuel supply which can be in any form suitable to allow pumping through the apparatus (1). In this example the fuel supply means (8) is a tank. The power means (9) can be used to activate and operate any of the components of the apparatus (1) including for example the UV light means (2) and the pumping means (4). The housing as shown in figures 2-5 is just one example of how the components of the apparatus can be arranged.

Other components either as part of the apparatus (1) and/or those that can be operatively connected to the apparatus (1) can include a pressure switch (10) and a filter means (11). As shown in figure 2 and 3 the filter means (1)1 is fluidly connected to an inlet means (12) whereby any large impurities such as for example solids, leaves dirt etc can be captured and removed before being treated by the apparatus (1). In this example the filter means (11) can be placed with the fuel tank.

The components of the apparatus can be supported by a support means (13) with or without the housing. The support means can include any structure that can support the components such as brackets, frames or panels. A manifold (14) can be used to provide flexibility in selection of any route through more than one filter means (11) or microbial contamination agitating means or UV light means.

The pressure switch (10) is there to protect the apparatus, noting any drop in pressure in fuel flow. For example if the fuel coming from the tank (8) is so contaminated, it either blocks the line or the filter or both. This blockage will cause a reduced or elimination of flow or reduction in pressure, which could cause the UV light bulb (21) to potentially over-heat or the pump (4) to burn out to cause failure or ignition danger. The pressure switch (10) senses any reduction in pressure (flow) and will turn the system off to prevent any such failure or danger.

### Sterilizing means (2) -UV light means with at least one light tube

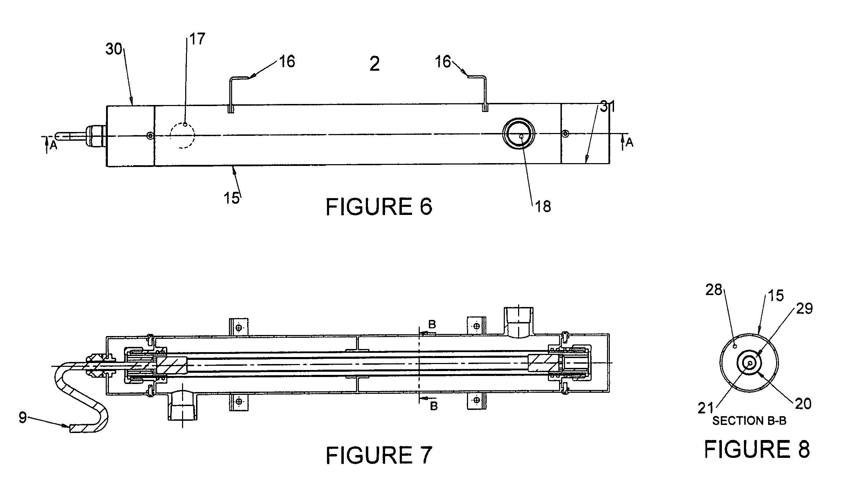

The UV light means (2) as shown in close up in figures 6-9 includes an elongate outer cylindrical housing called a first cylindrical or tubular housing (15) having mounting means (16) and inlet (17) at the bottom and outlet (18) at the top plus ends (19) and (20). Located within housing (15) there is another cylindrical housing or inner housing ie a second cylindrical or tubular housing (25) having ends (26 & 27) whereby there is an enclosed space (28) there-between for the fuel to move through during use of the apparatus (1). Within but spaced from the second cylindrical housing (25) there is removably fixed at the ends (26 & 27) of housing (25), a UV light (21).

The UV light (21) is in the form of a tubular light and is spaced from the second cylindrical housing (25) to form another enclosed space (29) but is anchored or supported at, at least one end as in figure (13) or both ends as shown in figure 7. The second cylindrical housing (25) is formed as a Teflon tube which is adapted to allow the UV light to pass through to treat the fuel passing through the outer enclosed space (28). The UV light means is not required to contact the fuel but to allow the fuel to travel past the UV light at a certain distance whereby the UV light sterilizes the fuel accordingly.

The microbial contamination agitating means (3) can be located behind the control means (7) but fluidly between the inlet (17) of the sterilizing means (2) and the pumping means (4).

Operatively coupled to an entry end (19) of first cylindrical housing (15), there is power means (9) to activate and operate the UV light (21) so that power means (9) as shown in figure (7) passes through the ends of both cylindrical housings (15) and (25) to be joined to the tubular light (21). First cylindrical housing (15) and second cylindrical housing (25) are fluidly sealed to allow the fuel to pass through without leaking. First cylindrical housing (15) combined with the second cylindrical housing (25) can be formed as end portions (30 & 31) with a middle portion (32). Middle portion (32) can also be formed in two con-joined halves as shown which are joined at the middle but are fluidly connected. Both end portions (30 & 31) are formed as separate fluidly sealed enclosed spaces (32) from a main inner fluidly sealed enclosed space (28).

If viewing the orientation of the housing in figure 2-5, there is an upper portion at the top of the page and a lower portion at the bottom of the page. The fuel is pumped into the sterilizing means (2) at a lower portion (below the light tube) at inlet (17) to fill up the lower portion of space (28) and circulate then upwards to an upper portion of the space (28) (above the light tube) of means (2) and then out of the sterilizing means (2) out of outlet (18) so that the sterilizing means (2) is fully loaded or filled and charged with fuel to maximize the efficiency of circulation past the UV light means (21) so that no hot spots are created on top of the light means especially if the fuel is pumped from the upper portion first.

Hot spots can occur with gravity feed of the fuel from the top of the sterilizing means (2). The apparatus is designed such that during circulation of the fuel there is no empty portion for fuel in the space (28) (but not in the inner housing (29)) which can cause problems of hot spots and possible ignition and explosive danger.

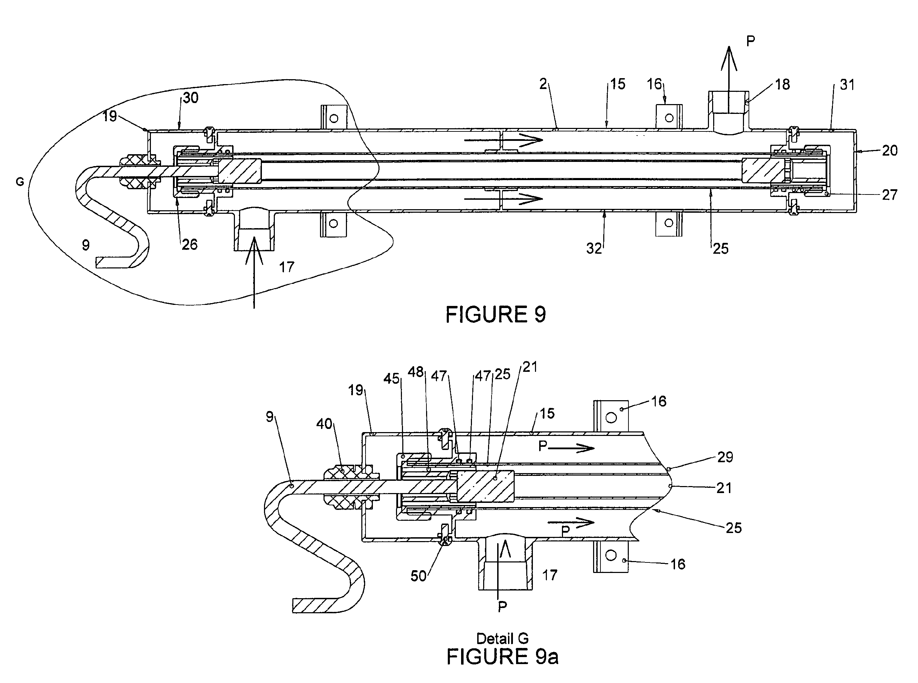

As shown in figure 9 the power means is sealed to housing (15) by a seal (40). The ends of the UV light is supported and sealed to the inner cylindrical housing (25) and to the ends of the end portions (30 & 31) and to middle portion (32), with a fixing means in the form of a UV light brush nut (45) threadably coupled to a male threaded base portion (46) which is formed as part of the end of the middle portion of the inner cylindrical housing (25). Between the male threaded base portion (46) and the end of the UV light tube end and cable connection portion there can be O rings (47) and a vented UV light bush (48). The O rings seal (47) between the male portion (46) and second cylindrical housing (25), to prevent fuel leakage while the venting is between the male portion (46) and the end of the UV light (21). Also shown in figure 9, there is a fixing means (50) allowing for removal of each end portion (30) and (31) to enable disconnection of and replacement of the UV light (21).

### Microbial contamination agitating means

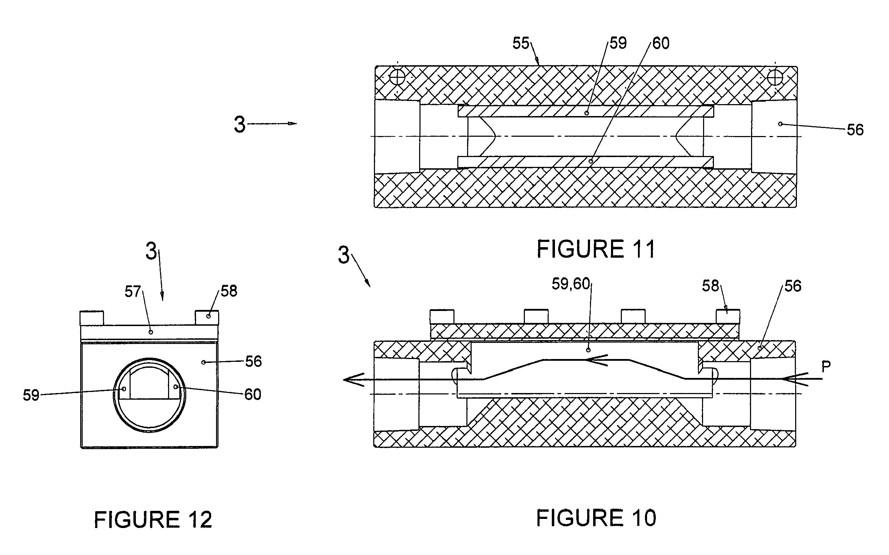

The microbial contamination agitating means (3) as in figure 1 and in close up in figures 10-12 includes a rectangular or elongate housing (55) including an elongate tubular space (56) therein and there through, which is constructed and adapted to allow fuel under pressure to pass through. Housing (55) includes an opening means (57) in the form of a removable lid with fixing means (58) which can be screws for example. Within the enclosed space (56) of the microbial contamination agitating means (3) there are located magnet means in the form of at least one magnet (59) and (60) which can be located in the middle portion of the passage way.

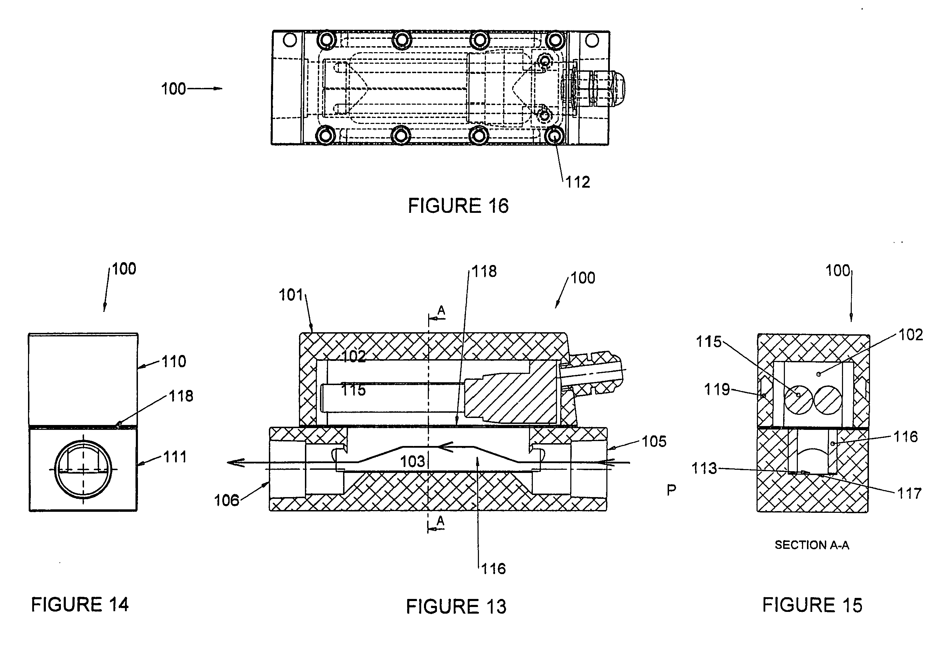

### Figures 13-16 - Combined sterilizing means and agitating apparatus

These figures show another validation of the fuel impurity reduction apparatus whereby the sterilizing means (2) is combined with the microbial contamination agitating means (3) in a sterilizing and microbial contamination agitating apparatus (100). This combination makes it possible to install a portable unit to any vessel or vehicle existing pump and filtration whereby any existing or new vehicle or vessel with re-circulating fuel technology can benefit from this installation. For example, this combination could be fitted to the common rail diesel engine in a land cruiser. This combination can easily be fitted to any supply line to the engine to continuously dry out the fuel which like the other apparatus of the present invention, can improve fuel quality and economy and engine performance.

Means (100) includes a housing (101) which as view with respect to figure 13 has an interior space and an outside surface. Housing (101) includes a box shape though this shape is not limited only to this shape. Housing (101) has a first or an upper portion (110) removably attached to a second or lower portion (111) by fixing means (112) which form separate enclosed spaces (102) and (103). Housing (101) has an inlet means (105) and outlet means (106) whereby in use fuel can enter the housing (101) through inlet means (105) and exit the housing through the outlet means (106). Both housings can be separately formed and joined or formed together or as one with separate compartments or recesses and they can be in other orientations other than be upper and lower.

In this example of apparatus (100), the first portion (110) can house a sterilizing means in the form of a UV lighting means (115) which produces UV light when activated. UV lighting means includes at least one elongate light bulb (in this example there can be two as seen in figure 15) which is fixed at one end to the housing (101) whereby the other end of the bulb is protruding into space (102) in the first portion (110).

The second portion (111) is designed to house an microbial contamination agitating means in the form of a passageway having magnetic means (116) therein and at one end of passage way (117) there is located the inlet (105) and at the other end (106). Separating the upper portion (106) and the lower portion (106) there is a fluid sealing means (118) which allows UV light there through to shine through and sterilized the passageway (117) which can have fluid or fuel being pumped through.

The fluid sealing means (118) can include a Teflon material separately or integrally combine with a gasket. Also shown is a venting means (119) which is formed as part of the first portion (110) which is designed to allow any heat built up by the bulbs of the sterilizing means (115) to be vented out of the housing. Also as shown the passage way (117) can be tortuous to force the fluid as close as possible to the light from the sterilizing means (115) to allow the fuel to be treated accordingly and at the same time go past the magnetic means (116) (to include magnets) and through a magnetic field and out of outlet (106) as shown by flow path line P in figure 13.

The magnets are shown as being placed in the middle of the passageway but can be placed elsewhere in the passage way, The shape and size of the magnets and the passageway can also be varied.

Other components as included in the first apparatus are also included in this apparatus (100) ie the pumping means and pressure switch. Any of the described apparatus can have branching means such as at least one manifold to divert flow as required. Also any valve means can also be used.

Any of the components of all apparatus as described in this specification, such as the sterilizing means, microbial contamination agitating means, pumping means, filter means, manifold, or valve means etc can be fitted and supported by the support means (13) within the housing or have no housing or be fitted directly to a housing.

Method of treating fuel by carrying out the following steps of:
- initiate control means - pumping begins and sterilizing means (2) in the form of a UV light (21) is on
- fuel is supplied in a fuel supply means or tank
- fuel then goes through the filter means (11) to remove any obvious contaminants
- pressure switch (10) operates to monitor pressure in the fuel line (5) to protect the pumping means (4) and UV light 21
- pumping means (4) pumps fuel under pressure from the filter means (11) to the microbial contamination agitating means (3)
- fuel then passes up into a lower portion of the UV light means (21) and through an upper portion of the UV light means (2)
- fuel passes into outer enclosed space (28) of the UV light means (2)
- fuel passes the UV light tube (21) through both middle portions (32) to be treated by reducing any impurities and
- treated fuel then passes out of the UV light means outlet (18)

To those skilled in the art to which the invention relates, many changes in construction and widely differing embodiments and application of the invention will suggest themselves without departing from the scope of the invention as defined in the appended claims. The disclosures and the descriptions herein are purely illustrative and are not intended to be limiting.

### Advantages

a) Simple installation
b) Modest cost
c) Straight forward manufacture
d) At least reduces fuel impurities
e) Assists in fuel economy
f) Dependable performance
g) Enhances fuel combustibility, thus increasing performance.
h) Microbial contamination agitating means and sterilizing mean can be combined in one separate housing unit.
i) Combination of sterilizing and agitating apparatus in a separate portable apparatus.
j) Combination can be installed to any common diesel recirculation engine system.
k) Treated fuel is dried out to enhance quality, economy and performance.
l) Apparatus can be adapted to include any number of components

### Variations

Throughout the description of this specification, the word "comprise" and variations of that word such as "comprising" and "comprises", are not intended to exclude other additives, components, integers or steps.

The fuel or liquid to be sterilized can be any hydrocarbon or organic non flammable fluid that can grow such contaminants. For example such fluids can be oil, diesel, cutting fluid but not petrol. All metalwork for piping or to allow the fuel to pass through and or be treated can be any coating or material that allows the fuel to be treated and not be contaminated or leak. For example the metalwork can be stainless steel.

The fuel means can be simply any pipe able to and having fuel supplied thereto which can be sourced from a tank locally or not. The filter means (11) can be placed within the fuel tank or be separate. There can be as many filters as required in any position in the apparatus as required. Some other types of filter are possible such as replaceable filters or back wash or reverseable filters.

The pumping means (4) can be any pump which can pump fluid such as a fuel or liquid hydrocarbon. The pump can be an electric type such as 12v or 24v in a DC type or 110v or 240v in an AC type pump.

The shapes of all of the housings for the microbial contamination agitating means and for the UV light means and the apparatus can be formed in any other shapes as well. Any number and shape of magnets are also equally possible

In another option (see figures 13-16) for the microbial contamination agitating means (3) and the sterilizing means (2) which can be formed as separate components of the fuel impurity reduction apparatus (1), they can also be formed as a combined apparatus within or connected to the other components. For example the sterilizing means (2) can have a microbial contamination agitating means (3) bolted directly to on top of the housing of the sterilizing means (2). This combination can also be directly fitted to a body of fuel such as in a tank to be protruding in a cantilever manner there within.

### Figure 17

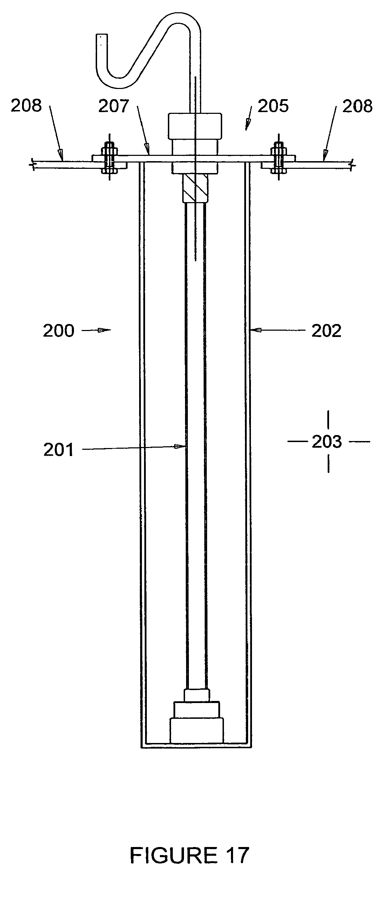

In yet another option there can be another apparatus called a UV apparatus (200) which includes UV light means (201) combined with a UV support means (202) whereby the UV support means allows the UV light means to be directly supported within the fuel (203) (e.g, in a tank). The UV light means (201) can be supported at least at one end (205) of the UV light (201) in a cantilevered support manner with the other end (206) of the light being unsupported by the tank but only supported by the other end of the support means as shown in figure 17. This support means (202) is spaced from the UV light and includes an end support plate (207) fixable to the tank (208) and the rest of the support can be in the form of a frame with or without a Teflon ™ tube or any means that seals the UV light directly from the fuel and yet allows the UV light there-through to function as required.

The manifold is a convenient device to allow for branching of various fluid flows and can be placed in the fluid flow as required. For example to allow control of the fluid flow into different filters or to divert into microbial contamination agitating means or not. Manual or automatic controllable valves can be used such as manual lever valves. The power means (9) can be mains or portable such as solar or battery.

### Figures 18-21

There is an apparatus (300) which is similar to that shown for figures 1-5 except that in this apparatus (300) there are multiple components such as four UV light means (302) in a vertical row, two filters with one being a removable filter (308) and the other being a back purge filter (309), isolating valves (304), fuel pumps (305), control means (307), support means (303) and housing (306). Similarly to the other apparatus the fuel to be treated is pumped through the filters (308) and (309) and then to through the valves which can be located in a manifold type structure and then through the UV light means (302) and then out or back for further treatment.

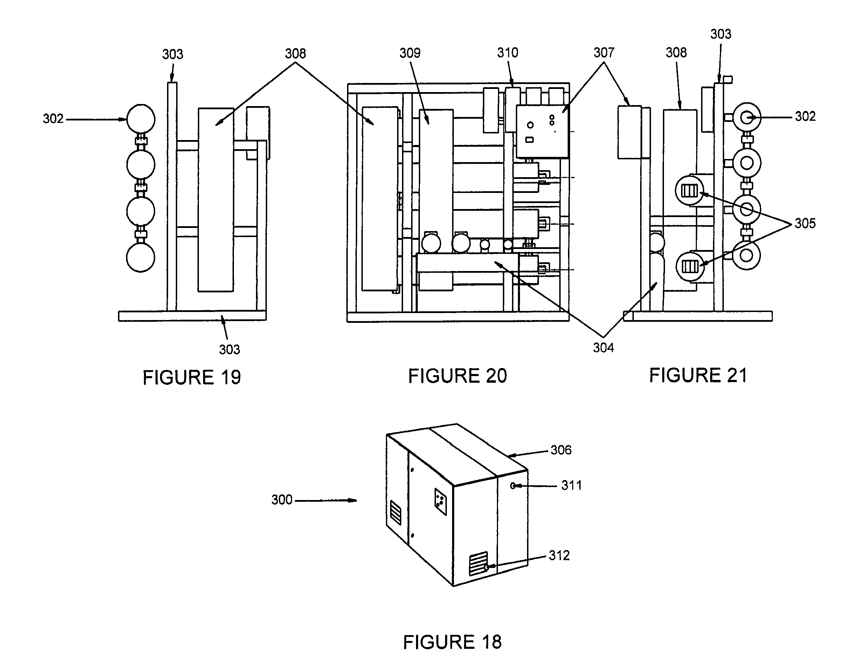

Extra filters can be used where required or the existing filter at least one, can be located within the apparatus (300) as required. Also UV controls (310) can be used to select which UV light means (302) can be used for a particular treatment and though two pumps (305) are shown, one can be used as a spare. Microbial contamination agitating means having magnetic means though not shown can also be used if desired. The housing (306) as shown in figure 18, though useful is not essential and the housing does show on one side of the housing, an outlet (311) positioned at the top of the housing close to the UV light means and an inlet (312) at the base of the housing (306). Grills are also shown in figure 18 which can be included to vent the inside of the housing. The support means (303) can be 50mmx500 RHS frame.

It will also be understood that where a product, method or process as herein described or claimed and that is sold incomplete, as individual components, or as a "kit of Parts", that such exploitation will fall within the ambit of the invention. The apparatus can be formed from a bank of horizontal UV light means, the filters can be vertical or horizontal and in multiple formation, multiple pumps and microbial contamination agitating means or linked fixed or portable apparatus.

It will of course be realised that while the foregoing has been given by way of illustrative example of this invention, all such and other modifications and variations thereto as would be apparent to persons skilled in the art are deemed to fall within the broad scope and ambit of this invention as is hereinbefore described.

Some other examples of variations of the apparatus of the present invention:
1. Include inlet to a first filter then to manifold and then to UV light means and from UV light means to the manifold or from other filter to the manifold, to then go to UV light means and out.
2. Another example (as being built at Rosebank Road) is two filter means, a stack of horizontal UV light means, manifold, gate valves, control means and microbial contamination agitating means.
3. Yet another example is having two pumps (one an emergency spare) with fuel pumped to a microbial contamination agitating means (with magnets) and then to a manifold (from either pump) and then to UV light means and then out.
4. Portable example including two horizontal UV light means, microbial contamination agitating means, filter valves and pump means whereby fuel is pumped when valve opened, to the microbial contamination agitating means, then to filter means and then to UV light means and out a valve outlet. This componentry can be supported by a vertical wall.

## Claims

1. A fuel impurity reduction apparatus to treat fuel including a sterilizing means, microbial contamination agitating means, pumping means, and fuel line, operatively interconnected together to a supply of fuel whereby the fuel is pumped under pressure to and through the microbial contamination agitating means and past the sterilizing means which includes a UV light means, and the microbial contamination agitating means includes a passageway with magnetic means therein, such that the fuel is treated to reduce the incidence of microbial contamination wherein the fuel is pumped from a lower end of the sterilizing means to an upper end of the sterilizing means so that the sterilizing means is always completely surrounded by fuel when the sterilizing means is activated.

2. The fuel impurity reduction apparatus as claimed in claim 1 wherein the apparatus includes a pressure switching means which is adapted to sense or monitor any reduction in pressure of the flow to enable the flow to be stopped, a manifold to allow a choice of filter means, microbial contamination agitating means or UV light means.

3. The fuel impurity reduction apparatus as claimed in claim 2 wherein, the UV light means includes a housing including an outer housing for the flow of fuel therein and an inner housing to hold a UV light means therein but separated from the flow of fuel, the outer housing includes a middle portion and end portions whereby the fuel only passes through the middle portion and end portions are fluidly sealed to not allow fuel therein and allow for the electrical connection of power means to power the UV light means.

4. The fuel impurity reduction apparatus as claimed in claim 3 wherein, the fuel to be treated enters a lower end of one end of the middle portion of the outer housing and passes along the middle housing past the UV light means, to reach the opposite end of the middle portion and then exit there from, and the UV light means is mounted above the pumping means whereby the outlet from the UV light means is located at the top of the UV light means.

5. The fuel impurity reduction apparatus as claimed in claim 4 wherein, the apparatus includes a housing to house the pumping means, the UV light means and microbial contamination agitating means.

6. A method of treating fuel to reduce fuel impurities when operating a fuel impurity reduction apparatus which includes including a sterilizing means, microbial contamination agitating means, pumping means, pressure switching means and fuel line, operatively interconnected together to a supply of fuel whereby the fuel is pumped under pressure to and through the microbial contamination agitating means and past the sterilizing means which includes a UV light means, and the microbial contamination agitating means includes a passageway with magnetic means therein such that the fuel is treated to reduce any microbial contamination in the fuel wherein the fuel is pumped from a lower end of the sterilizing means to an upper end of the sterilizing means so that the sterilizing means is always completely surrounded by fuel when the sterilizing means is activated, wherein the steps of the method include:
- pumping begins and sterilizing means is on;
- fuel then goes through the filter means;
- pressure switch operates to monitor the pressure in the fuel line;
- pumping means pumps fuel under pressure from the filter means to the microbial contamination agitating means
- alternatively the fuel can be pumped through another microbial contamination agitating means;
- fuel passes up into and through the sterilizing means;
- fuel passes into outer enclosed space of the sterilizing means;
- fuel passes the sterilizing means through both middle portions to be treated to reduce impurities and
- treated fuel then passes out of the sterilizing means outlet.

7. A microbial contamination agitating and sterilizing apparatus for treating fuel to reduce impurities, the apparatus includes a housing including a sterilizing means in one portion and microbial contamination agitating means in another portion, the sterilizing means including a UV light means in a first housing and the microbial contamination agitating means including a second housing with a passage way for the fuel and magnetic means located within the passage way, to allow fuel to travel through the passageway past the magnets and UV light, to be treated wherein there is a sealing means which allows the fuel to be fluidly sealed within the second housing and also allows UV light to penetrate there through to treat the fuel by sterilizing and agitating the fuel.

8. The microbial contamination agitating and sterilizing apparatus as claimed in claim 7 wherein the first housing has venting means to dissipate any heat build up from the UV light means and the magnet means are located in a middle portion of the passageway adjacent to the UV light means.

9. A fuel impurity reduction device substantially as herein described with reference to the figures of the accompanying drawings.

10. A method of treating fuel to reduce fuel impurities substantially as herein described with reference to the figures of the accompanying drawings.

11. A microbial contamination agitating and sterilizing apparatus substantially as herein described with reference to the figures of the accompanying drawings.
